# EUROPÄISCHE PATENTSCHRIFT

(11) **EP 0 783 403 B1**
(45) Veröffentlichungstag und Bekanntmachung des Hinweises auf die Patenterteilung: **12.08.1998**
(21) Anmeldenummer: 95913037.8
(22) Anmeldetag: 18.03.1995
(51) Int. Cl.: B29C 41/14, B29C 41/22, A61F 6/04

(54) **VERFAHREN UND VORRICHTUNG ZUR HERSTELLUNG EINES PROPHYLAKTIKUMS**
CONDOM PRODUCTION PROCESS AND DEVICE
PROCEDE ET DISPOSITIF DE PRODUCTION DE PRESERVATIFS

(30) Priorität: 28.09.1994 DE 4434701
(43) Veröffentlichungstag der Anmeldung: 16.07.1997
(73) Patentinhaber: SCHOLL, Thomas, F-67000 Strasbourg (FR)
(72) Erfinder: SCHOLL, Thomas, F-67000 Strasbourg (FR)
(74) Vertreter: Söffge, Karen
(86) Internationale Anmeldenummer: DE9500374
(87) Internationale Veröffentlichungsnummer: WO9525622

(56) Entgegenhaltungen:
- WO-A-94/13231
- CH-A- 429 147
- DE-A- 1 566 365
- DE-A- 1 616 474
- DE-A- 4 130 220

## Beschreibung

Die Erfindung bezieht sich auf ein Verfahren und eine Vorrichtung zur Herstellung eines Prophylaktikums aus dünnwandigem elastischen Material (Latex). Insbesondere befaßt sich die Erfindung mit einer möglichen Vorrichtung zur Herstellung des Prophylaktikums gemäß des nach Anspruch 1 definierten Verfahrens.

Derartige Verfahren und Vorrichtungen zur Herstellung dünnwandiger Formkörper, insbesondere aus Naturkautschukverbindungen, sind im Stand der Technik bekannt. Bei den bekannten Verfahren zur Herstellung eines Prophylaktikums handelt es sich gewöhnlich um einen bzw. zwei Tauchvorgänge, wobei das Tauchwerkzeug, das in der Regel aus poliertem Metall, Porzellan, Holz oder Glas gefertigt sein kann, mit seiner gesamten Tauchlänge in ein mit einer Latex-Mischung gefülltes Tauchbecken langsam hineingetaucht und langsam wieder herausgeführt wird. Zwischen dem ersten Tauchvorgang und dem zweiten Tauchvorgang findet ein Trocknungsvorgang statt, indem das am Tauchwerkzeug und am aufgebrachten elastischen Material befindliche Wasser ausgedampft wird.

Ohne auf jeden einzelnen Verfahrensschritt beim bekannten Verfahren einzugehen, ist es mit einem derartigen Verfahren unmöglich, ein Prophylaktikum mit einem erfindungsgemäßen elastischen Ring aus ein und demselben elastischen Material herzustellen, das den Anforderungen, die an ein Prophylaktikum zur Empfängnisverhütung oder zum Schutze vor ansteckenden Krankheiten von den Gesundheitsbehördern gefordert wird, gerecht wird.

Mit den im Stand der Technik bekannten Verfahren und Vorrichtungen lassen sich zwar den oberen Teil des Prophylaktikums umschließende Mehrfachringe herstellen, jedoch nicht ein einzelner Ring, der ein Vielfaches an Masse der im Stand der Technik bekannten Ringe aufweist.

Eine weitere im Stand der Technik bekannte Form eines Prophylaktikums ist in der Druckschrift US-1,142,443 beschrieben. Hierbei handelt es sich um ein Prophylaktikum, das im oberen geschlossenen Teil im Längsquerschnitt eine gewisse Kontur aufweist, die der anatomischen Form des männlichen Gliedes angepaßt ist. Nachteilig an dieser anatomischen Form wirkt sich der Umstand aus, daß, obwohl die anatomische Form gegeben ist, kein Haltepunkt entlang der Innenwand des Prophylaktikums vorhanden ist, der ihm einen sicheren und angenehmen Halt verleiht.

Ferner ist es mit den im Stand der Technik bekannten Herstellungs-Tauchverfahren unmöglich, ein mehrfarbiges Prophylaktikum herzustellen, bei dem die Farbe nicht in einem nachträglichen Arbeitsgang auf die Außenfläche des Prophylaktikums aufgebracht werden muß, was letztlich beim Gebrauch einen störenden Faktor darstellt.

Daher ist es Aufgabe der vorliegenden Erfindung, ein Prophylaktikum bereitzustellen, das im vorderen Teil einen Ring aus elastischem Material aufweist und bei Bedarf mehrfarbig hergestellt werden kann, wobei das Prophylaktikum fehlerfrei im Material ist und den Anforderungen der Prüfbehörden entspricht.

Diese Aufgabe wird erfindungsgemäß durch die in den Hauptansprüchen befindlichen kennzeichnenden Merkmale gelöst.

Entsprechend dem Oberbegriff des erfindungsgemäßen Verfahrens ist dieses darauf gerichtet, daß das Tauchwerkzeug bei mehr als einem Tauchvorgang unterschiedlich tief in das flüssige aufzubringende elastische Material eingetaucht wird, wobei es bevorzugt darauf ankommt, daß die erste Tauchtiefe wesentlich kürzer ist als die darauffolgenden Tauchtiefen und nach jedem Tauchvorgang eine Wärmebehandlung des Tauchwerkzeugs mit dem aufgebrachten elastischen Material (Latex) erfolgt.

Die zu dem erfindungsgemäßen Verfahren zur Herstellung eines Prophylaktikums gehörende erfindungsgemäße Vorrichtung zeichnet sich im wesentlichen dadurch aus, daß sie drei Tauchbecken, in denen sich eine spezielle Mischung (Latex), im wesentlichen bestehend aus Naturkautschuk, befindet, aufweist, wobei das erste der drei Tauchbecken relativ zum Endlos-Förderband wesentlich tiefer als die beiden anderen Tauchbecken liegt und zwischen dem ersten und dem zweiten und dem zweiten und dem dritten Tauchbecken jeweils Trocknungsöfen vorgesehen sind, denen ein bestimmtes Temperaturprofil und Temperaturprogramm aufgeprägt wird, bei dem die maximale Temperatur etwa 70°C beträgt; und hinter dem dritten Tauchbecken zunächst ein Trocknungsofen, eine Bürstenvorrichtung und ein weiterer Trocknungsofen vorgesehen ist; und einen über die gesamte Länge des Endlos-Förderbandes sich erstreckenden Ofens aufweist, dem ein bestimmtes Temperaturprofil und Temperaturprogramm aufgeprägt ist, wobei die maximale Temperatur etwa 110°C nicht übersteigt.

Besonders wichtig bei der vorliegenden Erfindung ist die Beschaffenheit des erfindungsgemäßen Tauchwerkzeugs, das vorzugsweise ein Glaszylinder ist, der an einem Ende geschlossen ist. Im Bereich des geschlossenen Endes weist das Tauchwerkzeug in vorteilhafter Weise eine bestimmte Kontur mit einer Vertiefung (Graben) auf, die an der tiefsten Stelle eine erfindungsgemäße Ringnut aufweisen kann, deren Öffnungswinkel zwischen 60° und 120° variiert. Bei der Auslegung der Maße für die Ringnut ist es vorteilhaft, die Tiefe der Ringnut zwischen 0,3 und 1,5 mm zu wählen. Neben verschiedenen Ausführungsformen des erfindungsgemäßen Tauchwerkzeugs erscheint die Ausführungsform am vorteilhaftesten, bei der sich seitlich der Ringnut zwei kegelstumpfförmige Abschnitte anschließen, deren Mantelflächen leicht ballig ausgebildet sind.

Am oberen, geschlossenen Ende des gläsernen Tauchwerkzeugs befindet sich ein besonders vorteilhaftes und erfindungsgemäßes Reservoirteil, das keulenartig ausgebildet ist, wobei das dünnere Ende der Keule an den Glaskolben anschließt und das obere größere Keulenende den Abschluß des Reservoirteils bildet. Die vorteilhaften Maße für das Reservoirteil sind im oberen breiteren Keulenteil etwa 12 mm und im unteren schmaleren Reservoirteil 4 bis 10 mm, wobei die Lange des Reservoirteils etwa 16 mm beträgt.

Weitere erfindungswesentliche Merkmale sind den Unteransprüchen zu entnehmen.

Im nun folgenden wird die Erfindung anhand von Zeichnungen näher beschrieben. Es zeigt:
- Fig. 1: eine schematische Darstellung der nach dem erfindungsgemäßen Verfahren arbeitenden Vorrichtung (1);
- Fig. 2: die Seitenansicht eines erfindungsgemäßen Tauchwerkzeugs (14) aus Glas;
- Fig. 3: eine geschnittene Teilansicht der Vertiefung mit der erfindungsgemäßen Ringnut (24);
- Fig. 4: eine Teilansicht des Tauchwerkzeugs (14) mit einem gebildeten Rollring (20) am Ende der Tauchschichten;
- Fig. 5: zwei Seitenansichten eines erfindungsgemäßen weiteren Tauchwerkzeugs (14) aus Glas;

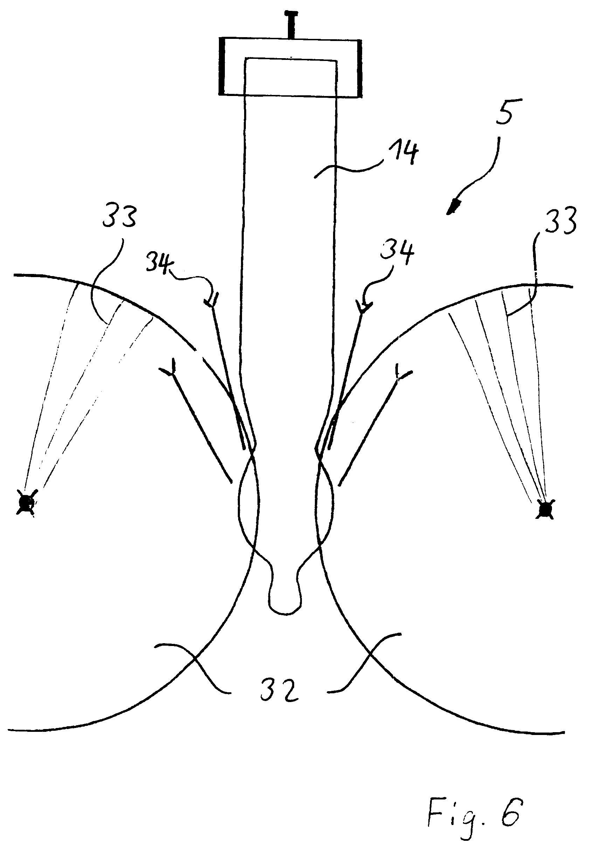

- Fig. 6: das Tauchwerkzeug (14) mit der schematisierten Abstreifvorrichtung (5).

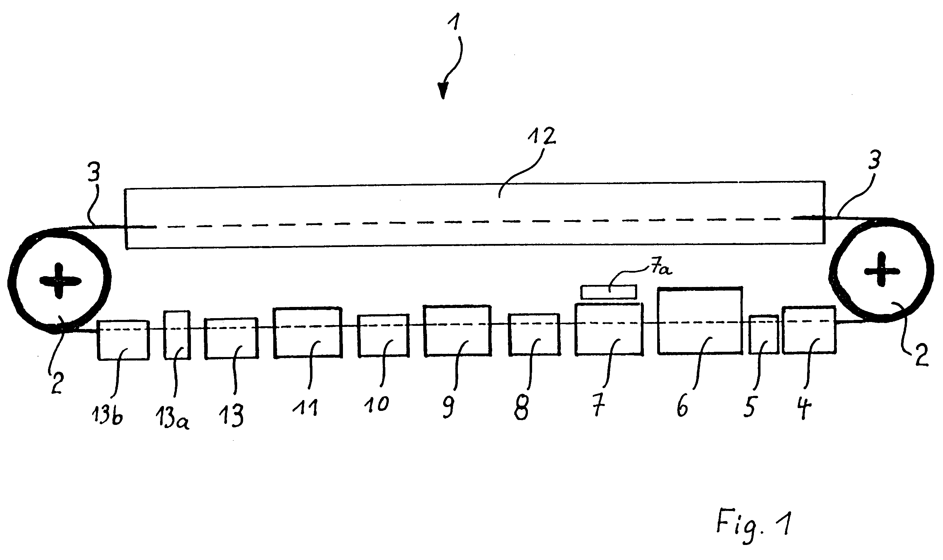

In Fig. 1 ist eine schematische Darstellung der erfindungsgemäßen Vorrichtung zur Herstellung eines dünnwandigen Prophylaktikums, das in mehreren Tauchvorgängen hergestellt wird, gezeigt. Die Vorrichtung 1 besteht aus einem Endlos-Förderband 3, das über zwei Rollen 2 geführt ist und von diesen mit der geforderten Fördergeschwindigkeit angetrieben wird. An dem Endlos-Förderband 3 sind die in dieser Zeichnung nicht gezeigten erfindungsgemäßen Tauchwerkzeuge, die weiter unten näher beschrieben werden, befestigt.

Im nun folgenden werden die wesentlichen Verfahrensschritte des erfindungsgemäßen Verfahrens bzw. Herstellungsprozesses im einzelnen beschrieben.

Die an dem Endlos-Förderband 3 befestigten Tauchwerkzeuge mit den fast fertigen Prophylaktika gelangen über die Rollen 2 in ein Waschbecken 4, das die wichtige Aufgabe hat, das aufgebrachte Latex-Material aufzuquellen, um bei einem weiteren Arbeitsgang mittels einer Abstreifvorrichtung 5 abgerollt zu werden und dann in einem nicht gezeigten Sammelkorb aufbewahrt werden.

In einer weiteren Bürstenvorrichtung 6 befinden sich drei Bürsteneinheiten, die einerseits dafür sorgen, daß keine hängengebliebenen Prophylaktika oder andere Rückstände an den Tauchwerkzeugen zurückbleiben und andererseits eine reinigende Wirkung ausüben.

Nach dem Sauberbürsten in der Bürstenvorrichtung 6 werden die Tauchwerkzeuge 14 direkt in das erste Tauchbad 7, das mit einer Latexmischung gefüllt ist, eingebracht. Wichtig ist hierbei, daß die Tauchwerkzeuge 14 eine bestimmten Temperatur haben, die sich als Mittelwert über die gesamte Förderbandlänge in den einzelnen Bearbeitungsschritten herausgestellt hat. Vor dem ersten Tauchvorgang im Tauchbecken 7 ist dafür Sorge zu tragen, daß die Oberfläche der Tauchwerkzeuge 14 nicht vollständig trocken ist, sondern einen leichten Feuchtigkeitsgehalt aufweisen. In dem ersten Tauchbecken des hier beschriebenen Ausführungsbeispiels wird das jeweilige Tauchwerkzeug 14 nur mit seinem konturierten Teil 17 ca. 100 mm in das Tauchbad eingetaucht. Infolge einer erfindungsgemäßen Schiene 7a, die oberhalb des Tauchbeckens 7 angeordnet ist, wird das Tauchwerkzeug 14 einerseits aus seiner Senkrechtlage in eine Schräglage gedrückt, und andererseits wird das Tauchwerkzeug 14 durch Reibschluß in Rotation versetzt. Die Rotation des Tauchwerkzeugs 14 dauert während des gesamten Tauchvorgangs an. Während einer kurzen Zeit durchläuft das Tauchwerkzeug 14 mit der vorgegebenen Tauchlänge das Latex-Bad und wird dann allmählich aus dem Bad herausgeführt. Die Tauchtiefe im ersten Tauchbecken 7 wird beim ersten sogenannten Spitzentauch dadurch erreicht, daß das gesamte Tauchbecken 7 insgesamt tiefergestellt wird. Das Eintauchen der Tauchwerkzeuge 14 geschieht in Schräglage, und das Austauchen der hängenden Tauchwerkzeuge 14 erfolgt in senkrecht zur Oberfläche des Tauchbades stehender Lage. Beim ersten sogenannten Spitzentauch tauchen die Tauchwerkzeuge 14 etwas später in das Latex-Bad ein und etwa 1 m vor dem Ende des Tauchbeckens 7 wieder aus dem Latex-Bad heraus.

Damit im Anschluß an den Spitzentauch eine unmittelbare Trocknungsphase einsetzen kann, wird die Trocknungsvorrichtung 8 etwa 1 m über dem Latex-Tauchbecken 7 vorgeschoben angebracht, so daß die benetzten Tauchwerkzeuge 14 in Waagrechtstellung in den ersten Trocknungsofen 8 einfahren können. Die Trocknung in dieser Trocknungsvorrichtung 8 bezieht sich lediglich auf die benetzte Spitze des Tauchwerkzeugs 14, wobei die Wärme so gerichtet ist, daß lediglich die Werkzeugspitze erwärmt wird.

Unmittelbar nach Beendigung des Tauchvorganges, d.h. sobald das Tauchwerkzeug 14 die Oberfläche des flüssigen Materials verlassen hat, wird das Tauchwerkzeug 14 durch einen am offenen Ende des Tauchwerkzeugs befestigten Mechanismus in Rotation versetzt und gleichzeitig um 90° gedreht in die waagrechte Lage gebracht. In dieser waagrechten Lage durchläuft das mit elastischem Material benetzte Tauchwerkzeug 14 die erste Trocknungsstufe im Trocknungsofen 8. Dieser Trocknungsofen 8 ist mit einem bestimmten Temperaturprofil beaufschlagt, dessen maximale Temperatur ca. 70° nicht übersteigt. Während dieser ersten Trocknungsstufe im Trocknungsofen 8 wird das auf das Werkzeug 14 aufgebrachte elastische Material (Latex) einer gewissen Aushärtung bzw. Befestigung unterzogen und andererseits für den zweiten Tauchvorgang im zweiten Tauchbecken 9 vorbereitet und eventuelle Wasserrückstände ausgedampft.

Die nach dem ersten Tauchvorgang beschichtete Oberfläche des Werkzeugs 14 im vorderen Teil 17 des Tauchwerkzeugs 14 ist nun so präpariert, daß ein zweiter Tauchvorgang angeschlossen werden kann, in dem das Tauchwerkzeug 14 bis zum Ende der tatsächlichen Länge des Prophylaktikums, verkürzt um den Materialverbrauch zur Bildung eines Rollrings 20, eintaucht. Das Eintauchen erfolgt verhältnismäßig schnell im Vergleich zum Herausführen. Unmittelbar nachdem das Tauchwerkzeug 14 die Oberfläche des Tauchbades (Latex) verlassen hat, wird es wieder in Rotation versetzt und in eine waagrechte Position gebracht, in der es die zweite Trocknungsstufe im Ofen 10 durchläuft. Der Trocknungsofen 10 ist ebenfalls mit einem bestimmten Temperaturprofil beaufschlagt, dessen maximale Temperatur etwa 70°C beträgt. Die Durchlaufgeschwindigkeit wird durch die Fördergeschwindigkeit des Endlos-Förderbandes 3 bestimmt und ist während eines Produktionszyklus konstant. Die Verweilzeit richtet sich demgemäß nach der Länge des jeweiligen Trocknungsofens.

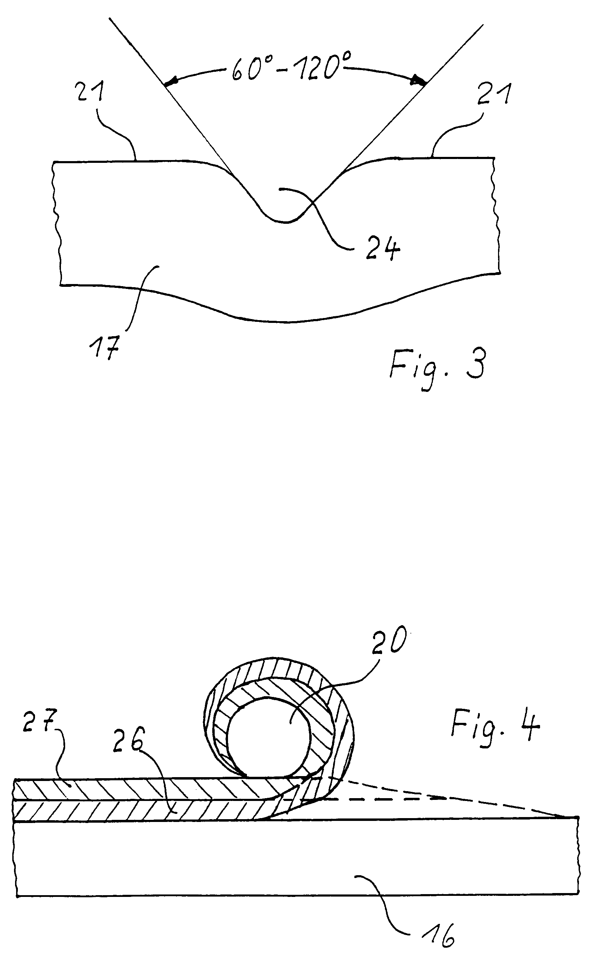

Nachdem das Tauchwerkzeug am Endlos-Förderband 3 den Trocknungsofen 10 verlassen hat, erfolgt der dritte und letzte Tauchvorgang im Tauchbecken 11. In diesem Tauchbecken 11 taucht das Tauchwerkzeug 14, wie bereits vorher beschrieben wurde, in gleicher Weise in das Tauchbecken 11 bis zu einer Länge ein, die knapp unter der Eintauchlänge des vorangegangenen Tauchvorganges im Tauchbecken 9 liegt. Im Anschluß an den dritten und letzten Tauchvorgang im Tauchbecken 11 erfolgt wiederum eine Trocknung im Trocknungsofen 13, die in ähnlicher Weise wie vorher beschrieben wurde, abläuft. Danach erfolgt die Bildung eines Rollrings 20 (Fig 4), der mit Hilfe rotierender Bürsten, die hier nicht näher gezeigt werden, erstellt. Nach der Herstellung des Rollrings 20 auf dem Tauchwerkzeug 14 in der Vorrichtung 13a durchläuft das Tauchwerkzeug eine weitere Trocknungsstufe 13b.

Nach der Herstellung des Rollrings 20 auf dem Tauchwerkzeug 14 durchläuft das Tauchwerkzeug mit dem bereits fertigen Prophylaktikum die letzte Wärmebehandlung im Wärmeofen 12, in dem zunächst ein Trocknungsvorgang einsetzt.

Der über die gesamte Länge der Vorrichtung am oberen Teil des Endlos-Förderbandes 3 sich erstreckende Ofen 12 ist mit einem ganz bestimmten Temperaturprogramm und einem bestimmten Temperaturprofil beaufschlagt, wobei die maximale Temperatur innerhalb des Ofens 12 110°C nicht übersteigt. Die Verweilzeit des fertigen Prophylaktikums richtet sich selbstverständlich wieder nach der Geschwindigkeit des Endlos-Förderbandes 3 und liegt typischerweise zwischen 6 cm/s und 8 cm/s, woraus eine Verweilzeit von ca. 9 Minuten resultiert. Nach Verlassen des Ofens 12 wird das fertige Prophylaktikum auf dem Tauchwerkzeug 14 über das Umlenkrad 2 in das vorher bereits erwähnte Laugenbad 4 getaucht, um ein Aufquellen des fertigen Prophylaktikums zu erzielen.

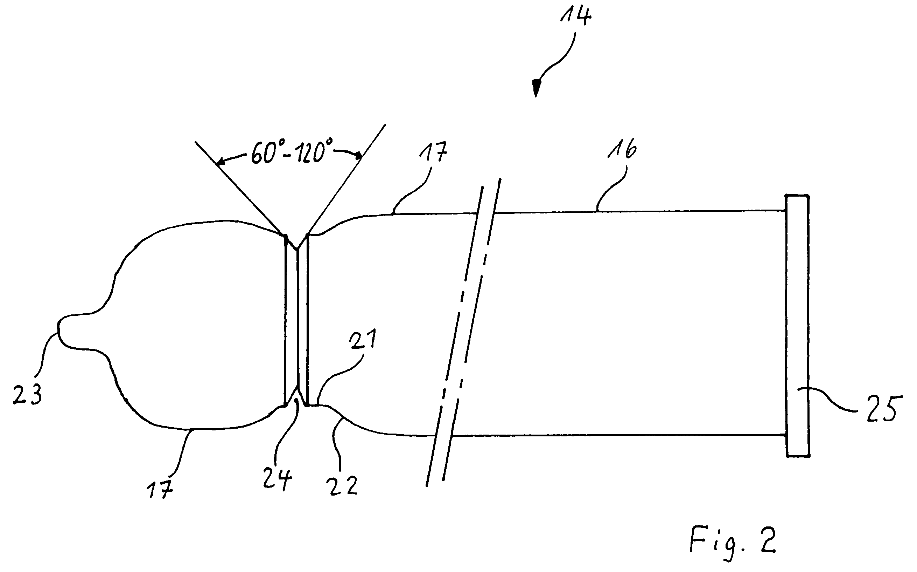

In Fig. 2 ist ein Tauchwerkzeug 14 in einer Seitenansicht dargestellt. Das Tauchwerkzeug 14 besteht in diesem Falle aus einem langgestreckten Glaszylinder (350 mm lang), der im wesentlichen in zwei Bereiche 16 und 17 unterteilt ist, wobei der Bereich 16 ein langgestreckter gerader Zylinder ist, an den sich in dieser Darstellung linksseitig ein geformter, am Ende geschlossener Teil 17 anschließt. Am offenen Teil 16 des Glaszylinders befindet sich ein Absatz 25, der dazu dient, das gesamte Tauchwerkzeug 14 an einer nicht näher erläuterten Haltevorrichtung am Endlos-Förderband 3 zu befestigen. Der geformte Teil 17 des Tauchwerkzeugs 14 weist an seinem linken Ende eine Ausbuchtung 23 auf, die etwa 15 mm lang ist und an seinem offenen Ende ca. 15 mm Durchmesser aufweist. Durch diese Ausbuchtung wird beim späteren fertigen Prophylaktikum das sogenannte Reservoir hergestellt. Der Übergang von der Ausbuchtung 23 zum aufgeweiteten vorderen Teil 17 erfolgt mit einem angemessenen weich verlaufenden Krümmungsradius. Der vordere aufgeweitete Teil 17 des Tauchwerkzeugs 14 weist ferner einen zylindrischen geraden Teil auf, der ca. 15 mm lang ist.

An den geraden Teil schließt sich eine Vertiefung 22 an, die praktisch eine Einengung des Durchmessers des Tauchwerkzeugs 14 darstellt. Etwa in der Mitte der Vertiefung 22 erstreckt sich über einen kurzen Bereich ein gerades Stück, das etwa 6 mm lang ist. In der Mitte dieser geraden, parallel zur Längsachse verlaufenden Vertiefung befindet sich eine Ringnut 24, bei der der Öffnungswinkel zwischen 60° und 120° variiert. Die Tiefe der Ringnut 24 richtet sich nach den Bedürfnissen des Anwenders des Prophylaktikums und liegt zwischen 0,3 und 1,5 mm. Die Querschnittsform der Ringnut 24 ist nicht a priori festgelegt, sondern richtet sich nach dem zu verwendenden elastischen Material. Beispielsweise kann eine im Querschnitt dreieckige Ringnut gewählt werden, die verhältnismäßig kleine Krümmungsradien an den Übergangsstellen zum flachen Teil der Vertiefung 22 aufweisen können (siehe Fig. 2). Eine andere denkbare Ringnut 24 ist in Fig. 3 dargestellt. Hier verlaufen die Übergänge von den flachen Teilen mit einem verhältnismäßig großen Krümmungsradius, wobei jedoch der Öffnungswinkel zwischen 60° und 120° erhalten bleibt.

Die Einarbeitung der Ringnut 24 erfolgt mit bekannten handwerklichen Mitteln und wird an dieser Stelle nicht näher beschrieben. Das gleiche gilt für die Erstellung des gesamten Tauchwerkzeugs 14 aus Glas.

Fig. 4 zeigt einen Teilausschnitt des Tauchwerkzeugs 14, auf das zunächst zwei Schichten 26, 27 des elastischen Materials (Latex) in den einzelnen Tauchbädern aufgebracht und anschließend mit Hilfe rotierender Bürsten zu einem Rollring aufgerollt wurde. Die rotierenden Bürsten greifen zunächst an der ersten Schicht 26 an, rollen diese zusammen mit der zweiten Schicht 27 zu einem Rollring 20 zusammen. Bei einer anschließenden Wärmebehandlung wird der Rollring mit seinen einzelnen Schichten 26 und 27 vernetzt, so daß er eine kompakte Einheit bildet.

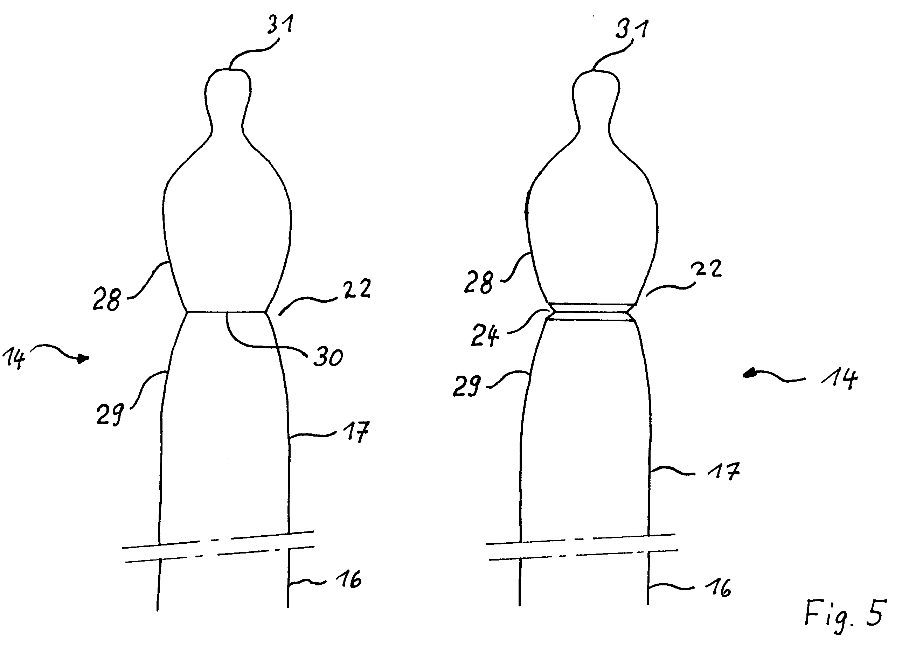

In Fig. 5 ist ein weiteres erfindungsgemäßes Ausführungsbeispiel des Tauchwerkzeugs 14 dargestellt. Das Tauchwerkzeug 14 besteht aus einem langgestreckten zylindrischen Teil 16, an den sich ein konturierter Teil 17 des Tauchwerkzeugs 14 anschließt.

Diese besonders wichtige Ausführungsform des Tauchwerkzeugs 14 bzw. des fertigen Prophylaktikums hat seine Besonderheit darin, daß einerseits die Vertiefung 22 durch zwei kegelstumpfartige Teile 28 und 29 gebildet wird. An der tiefsten Stelle, an der die beiden kleinen Deckflächen der Kegelstümpfe aneinanderstoßen, kann wahlweise eine Ringnut 24, wie vorher beschrieben, eingearbeitet werden oder aber die Ringnut entfällt. Für den Fall, daß die Ringnut nicht eingearbeitet ist, ist es von außerordentlicher Bedeutung, daß die Stoßstellen der Kegelstümpfe 28 und 29 einen scharfen ausgeprägten Knick 30 bilden. Bei entsprechendem Material kann dieser ausgeprägte Knick bzw. Rille 30 die Bildung des erfindungsgemäßen Ringes hervorrufen bzw. die erfindungsgemäße Ringnut 24 ersetzen. Ein weiteres wichtiges erfindungsgemäßes Merkmal dieser Ausführungsform ist die Balligkeit der Mantelflächen der Kegelstümpfe 28 und 29. Der Durchmesser der kleinen Deckflächen der Kegelstumpfe 28, 29 ist zwischen 16 und 30 mm zu wählen.

Entsprechend der vorliegenden Erfindung befindet sich am oberen Ende des Werkzeugs 14 ein sogenanntes Reservoir 31, das erfindungsgemäß in dieser Ausführungsform keulenartig ausgebildet ist. Besonders hervorzuheben sind dabei die Maße des Reservoirs 31, die am unteren schmalen Ende zwischen 4 bis 10 mm liegen und am oberen Ende 12 mm betragen. Die Länge des Reservoirs 31 ist ca. 16 mm.

Infolge der konturierten Form des Tauchwerkzeugs 14 ergeben sich beim Abstreifen des fertigen Prophylaktikums mit der Abstreifvorrichtung 5 erhebliche Schwierigkeiten, die mittels einer Abstreifvorrichtung 5 überwunden werden. Diese Abstreifvorrichtung 5 weist im wesentlichen gegensinnig rotierende Bürsten 32 auf, deren Haare 33 während der Rotation die Oberfläche des Tauchwerkzeugs 14 berühren und dadurch ein Abstreifen des Prophylaktikums bewirken. Problemlos ist der Abstreifvorgang jedoch nur im langgestreckten länglichen Teil des Tauchwerkzeugs 14, wohingegen sich im konturierten Teil des Tauchwerkzeugs Schwierigkeiten beim Abstreifen ergeben. Um diese Schwierigkeiten zu überwinden, sind seitlich des Tauchwerkzeugs 14 über den Bürsten 32 eine oder mehrere Düsen 34 angeordnet, die einen druckbeaufschlagten Wasserstrahl in Richtung des konturierten Teils des Tauchwerkzeugs 14 lenken. Dadurch ergibt sich ein müheloser Abstreifvorgang vom konturierten Teil des Tauchwerkzeugs 14.

## Patentansprüche

1. Verfahren zur Herstellung eines Prophylaktikums, wobei das Prophylaktikum aus einem Formteil aus dünnwandigem elastischen Material besteht, das in Tauchbädern auf ein Tauchwerkzeug äußerlich aufgebracht wird, und das Formteil aus einem zylindrischen Abschnitt (16) und einem geformten Abschnitt (17) besteht, und im oberen geformten Abschnitt (17) einen Ring aufweist, wobei das Tauchwerkzeug (14) mit seinem spitzen Bereich bei einem von mehreren Tauchvorgängen wesentlich unterschiedlich tief in das flüssige aufzubringende elastische Material (Latex) eingetaucht wird, wobei nach jedem Tauchvorgang eine Warmbehandlung erfolgt.

2. Verfahren nach Anspruch 1, **dadurch gekennzeichnet**, daß das Tauchwerkzeug (14) vor dem ersten Tauchvorgang von Rückständen gereinigt wird und die Oberfläche so präpariert wird, daß auf dem Tauchwerkzeug (14) niedergeschlagenes elastisches Material leicht abstreifbar ist.

3. Verfahren nach Anspruch 1, **dadurch gekennzeichnet**, daß beim ersten Tauchvorgang mit einem geformten Tauchwerkzeug (14) nur der geformte Abschnitt (17) in das erste Tauchbad (7) so eintaucht, daß mindestens die Vertiefung (22) vollständig in das erste Tauchbad (7) eintaucht.

4. Verfahren nach Anspruch 1 und 3, **dadurch gekennzeichnet,** daß das Tauchwerkzeug (14) beim ersten Tauchvorgang mit seiner Längsachse in einem bestimmten Winkel zur Oberfläche des elastischen aufzubringenden Materials um seine Längsachse drehend durch das Tauchbad (7) geführt wird.

5. Verfahren nach Anspruch 1, **dadurch gekennzeichnet,** daß das Tauchwerkzeug (14) mit dem aufgebrachten elastischen Material einem ersten Trocknungsvorgang (8) unterzogen wird.

6. Verfahren nach Anspruch 1, **dadurch gekennzeichnet,** daß nach dem ersten Trocknungsvorgang ein zweiter Tauchvorgang stattfindet, bei dem das Tauchwerkzeug (14) am weitesten in ein zweites Tauchbecken (9) eingeführt wird.

7. Verfahren nach Anspruch 1, **dadurch gekennzeichnet,** daß nach dem zweiten Tauchvorgang ein zweiter Trocknungsvorgang (10) durchgeführt wird.

8. Verfahren nach Anspruch 1, **dadurch gekennzeichnet,** daß nach dem zweiten Trocknungsvorgang ein dritter Tauchvorgang in einem dritten Tauchbecken (11) vorgenommen wird, bei dem das Tauchwerkzeug (14) nicht wesentlich kürzer als beim zweiten Tauchvorgang in ein drittes Tauchbecken (11) eingeführt wird.

9. Verfahren nach Anspruch 1, **dadurch gekennzeichnet**, daß nach dem dritten Tauchvorgang (13) eine Trocknung vorgenommen wird, im Anschluß daran mittels gegensinnig rotierender Bürsten (13a) ein Rollring (20) gebildet wird und im Anschluß daran eine Trocknung (13b) vorgenommen wird.

10. Verfahren nach einem oder mehreren der vorangegangenen Ansprüche, **dadurch gekennzeichnet,** daß das Tauchwerkzeug (14) während der einzelnen Trocknungsvorgänge zwischen den Tauchvorgängen ein bestimmtes Temperaturprogramm durchläuft, bei dem die Temperatur 70°C nicht übersteigt.

11. Verfahren nach einem der vorangegangenen Ansprüche, **dadurch gekennzeichnet,** daß die Trocknungszeit der einzelnen Trocknungsvorgänge zwischen den Tauchvorgängen etwa 1,5 Minuten dauert.

12. Verfahren nach Anspruch 1, **dadurch gekennzeichnet,** daß das Tauchwerkzeug (14) zusammen mit dem aufgebrachten elastischen Material im Anschluß an die Bildung des Rollrings (20) einer Wärmebehandlung gemäß eines bestimmten Temperaturprogramms unterzogen wird, bei dem die maximale Temperatur ca. 110°C nicht übersteigt.

13. Verfahren nach Anspruch 1, **dadurch gekennzeichnet,** daß das an einem Förderband (3) hängend befestigte Tauchwerkzeug (14) mit einer bestimmten Geschwindigkeit, die zwischen 6 cm/s und 9 cm/s variiert, durch die Tauchbäder (7, 9, 11) bewegt werden.

14. Verfahren nach Anspruch 1, **dadurch gekennzeichnet,** daß das Tauchwerkzeug (14) während der einzelnen Wärmebehandlungen um die Längsachse gedreht wird.

15. Verfahren nach Anspruch 14, **dadurch gekennzeichnet,** daß die Rotation des Tauchwerkzeugs (14) unmittelbar nach dem vollständigen Herausnehmen aus den Tauchbädern (7, 9, 11) beginnt.

16. Verfahren nach einem der vorangegangenen Ansprüche, **dadurch gekennzeichnet,** daß das Tauchwerkzeug (14) in waagerechter Stellung durch die Wärmebehandlungsvorrichtungen (8, 10, 12, 13, 13b) geführt wird.

17. Verfahren nach einem oder mehreren der vorangegangenen Ansprüche, **dadurch gekennzeichnet,** daß das fertige Prophylaktikum von dem Tauchwerkzeug (14) mittels gegensinnig rotierender Bürsten unter Zuhilfenahme mindestens eines druckbeaufschlagten Wasserstrahls (35) abgestreift wird.

18. Verfahren nach Anspruch 1 und 17, **dadurch gekennzeichnet,** daß der mindestens eine Wasserstrahl (35) einen bestimmten Winkel zur Längsachse des Tauchwerkzeugs (14) einnimmt und im vorderen Teil auf das Tauchwerkzeug (14) einwirkt.

19. Vorrichtung zum Herstellen eines Prophylaktikums, wobei das Prophylaktikum aus einem Formteil aus dünnwandigem elastischen Material besteht, das in Tauchbädern (7, 9, 11) auf ein Tauchwerkzeug (14) äußerlich aufgebracht wird und das Tauchwerkzeug (14) aus einem zylindrischen Abschnitt (16) und einem geformten Abschnitt (17) besteht und im geformten Abschnitt (17) eine Ringnut (24) und ein Reservoirteil (23, 31) aufweist, und das Tauchwerkzeug (14) an einem Endlos-Förderband (3) befestigt ist, **gekennzeichnet durch**
- drei Tauchbecken (7, 9, 11), in denen sich eine spezielle Mischung (Latex), im wesentlichen bestehend aus Naturkautschuk, befindet, wobei das erste (7) der drei Tauchbecken (7, 9, 11) relativ zum Endlos-Förderband (3) wesentlich tiefer als die beiden anderen Tauchbecken (9, 11) liegt;
- zwischen dem ersten und dem zweiten und dem zweiten und dem dritten Tauchbecken jeweils Trocknungsöfen (8, 10, 13) vorgesehen sind, denen ein bestimmtes Temperaturprofil und Temperaturprogramm aufgeprägt ist, bei dem die maximale Temperatur etwa 70°C beträgt; und
- hinter dem dritten Tauchbecken (11) zunächst ein Trocknungsofen (13), eine Bürstenvorrichtung (13a) und ein weiterer Trocknungsofen (13b) vorgesehen ist; und
- einen über die gesamte Länge des Endlos-Förderbandes (3) sich erstreckenden Ofen (12) aufweist, dem ein bestimmtes Temperaturprofil und Temperaturprogramm aufgeprägt ist, wobei die maximale Temperatur etwa 110°C nicht übersteigt;
- eine längs des Tauchbeckens (7) geführte Schiene (7a), die der Längsachse des Tauchwerkzeugs (14) eine bestimmte Schräglage verleiht und das Tauchwerkzeug (14) in Drehung versetzt;
- eine Abstreifvorrichtung (6), die gegensinnig rotierende Bürsten und einen auf den vorderen Teil des Tauchwerkzeugs (14) einwirkenden druckbeaufschlagten Wasserstrahl (35).

20. Vorrichtung nach Anspruch 19, **dadurch gekennzeichnet,** daß das Tauchwerkzeug (14) ein langgestreckter, an einem Ende geschlossener, vorzugsweise ein Glaszylinder ist, der an der Oberfläche im Bereich des geschlossenen Endes eine bestimmte Kontur mit einer Vertiefung (Graben) (22) aufweist.

21. Vorrichtung nach Anspruch 19 und 20, **dadurch gekennzeichnet,** daß die Vertiefung (22) sich über ca. 30 mm erstreckt, wobei die Vertiefung (22) etwa 50 mm nach dem geschlossenen Ende (23) des Tauchwerkzeugs (14) beginnt.

22. Vorrichtung nach Anspruch 20, **dadurch gekennzeichnet,** daß die Vertiefung (22) in der Mitte einen geraden Teil (21) aufweist.

23. Vorrichtung nach Anspruch 19 und 20, **dadurch gekennzeichnet,** daß in etwa der Mitte des geraden Teils (21) eine Ringnut (24) eingearbeitet ist, deren Öffnungswinkel zwischen 60° und 120° variiert.

24. Vorrichtung nach einem der vorangegangenen Ansprüche, **dadurch gekennzeichnet,** daß die Ringnut (24) keine scharfen Spitzen, Ecken und Kanten aufweist.

25. Vorrichtung nach einem der vorangegangenen Ansprüche, **dadurch gekennzeichnet,** daß die Tiefe der Ringnut (24) zwischen 0,3 und 1,5 mm variiert.

26. Vorrichtung nach einem der vorangegangenen Ansprüche, **dadurch gekennzeichnet,** daß die Förderbandgeschwindigkeit während eines Produktionszyklus konstant ist und rund 8 cm/s beträgt.

27. Vorrichtung nach Anspruch 19 und 20, **dadurch gekennzeichnet,** daß die Vertiefung (Graben) (22) sich über ca. 60 mm erstreckt, wobei die Vertiefung (22) etwa 40 mm nach dem geschlossenen Ende (23, 31) des Tauchwerkzeugs (14) beginnt.

28. Vorrichtung nach Anspruch 19, 20 und 25, **dadurch gekennzeichnet,** daß die Vertiefung aus zwei annähernd identischen konischen, kegelstumpfartigen Teilen (28, 29) zusammengesetzt ist, wobei die kleinen Deckflächen der Kegelstümpfe (28, 29) aneinanderstoßen, wodurch eine scharfe Trennlinie (30) gebildet wird.

29. Vorrichtung nach Anspruch 19, 20, 23 und 25, **dadurch gekennzeichnet,** daß die Trennlinie (30) eine Ringnut (24) aufweist.

30. Vorrichtung nach Anspruch 28, **dadurch gekennzeichnet,** daß die Mantelflächen der Kegelstümpfe (28, 29) ballig sind.

31. Vorrichtung nach Anspruch 19 und 20, **dadurch gekennzeichnet,** daß das Reservoirteil (31) am oberen Ende des Prophylaktikums keulenartig ausgebildet ist, wobei das schmalere Teil des Reservoirs (31) an den Glaskolben anschließt.

32. Vorrichtung nach einem der vorangegangenen Ansprüche, **dadurch gekennzeichnet,** daß der Durchmesser der Trennlinie (30) zwischen 16 und 30 mm variiert.

33. Vorrichtung nach Anspruch 31, **dadurch gekennzeichnet,** daß der Durchmesser des schmaleren unteren Teils des Reservoirs (31) zwischen 4 und 10 mm variiert.

34. Vorrichtung nach Anspruch 31, **dadurch gekennzeichnet,** daß der Durchmesser des breiteren oberen Teils des Reservoirs (31) 12 mm und die Länge des Reservoirs (31) 16 mm ist.

35. Vorrichtung nach einem der vorangegangenen Ansprüche, **dadurch gekennzeichnet,** daß die Farben des auf das Tauchwerkzeug (14) während des Tauchvorganges aufzubringenden elastischen Materials in den einzelnen Tauchbecken (7, 9, 11) unterschiedlich sind.

36. Vorrichtung nach einem der vorangegangenen Ansprüche, **dadurch gekennzeichnet,** daß die Abstreifvorrichtung zum Abstreifen des fertigen Prophylaktikums gegensinnig rotierende Bürsten (32) aufweist.

37. Vorrichtung nach Anspruch 19 und 36, **dadurch gekennzeichnet,** daß die Abstreifvorrichtung mindestens eine Wasserstrahldüse aufweist, deren druckbeaufschlagter Wasserstrahl im vorderen Teil des Tauchwerkzeugs (14) auf das Tauchwerkzeug einwirkt.

## Claims

1. Process for producing a prophylactic, whereby the prophylactic consists of a shaped part made of thin-walled, elastic material, which is externally applied to an immersion tool in immersion baths, and the shaped part consists of a cylindrical sections (16) and a shaped section (17) and has a ring in the top, shaped section (17), whereby the immersion tool (14) is immersed with its pointed zone in one of several immersion stages with a substantially different depth in the liquid elastic material (latex) to be applied, whereby a heat treatment takes place after each immersion stage.

2. Process according to claim 1, characterized in that the immersion tool (14) is cleaned prior to the first immersion stage from residues and the surface is prepared in such a way that elastic material deposited on the immersion tool (14) can be readily stripped off.

3. Process according to claim 1, characterized in that in the first immersion stage with a shaped immersion tool (14), only the shaped section (17) immserses in the first immersion bath (7) in a way such that at least the deepening (22) completely immerses in the first immersion bath (7).

4. Process according to claims 1 and 3, characterized in that the immersion tool (14), in the first immersion stage, is guided through the immersion bath (7) rotating around its longitudinal axis, with its longitudinal axis at a defined angle relative to the surface of the elastic material to be applied.

5. Process according to claim 1, characterized in that the immersion tool (14) with the applied elastic material is subjected to a first drying stage (8).

6. Process according to claim 1, characterized in that after the first drying stage, a second immersion stage takes place, in which the immersion tool (14) is immsersed farthest in a second immersion tub (9).

7. Process according to claim 1, characterized in that after the second immersion stage, a second drying stage (10) is carried out.

8. Process according to claim 1, characterized in that after the second drying stage, a third immersion stage is carried out in a third immersion tub (11), in which the immersion tool (14) is immersed in a third immersion tub (11) not substantially shorter than in the second immersion stage.

9. Process according to claim 1, characterized in that after the third immersion stage (13), a drying is carried out, a rolling ring (20) is subsequently formed by means of brushes (13a) rotating in opposite directions, and a drying (13b) is carried out thereafter.

10. Process according to one or several of the preceding claims, characterized in that the immersion tool (14), during the individual drying stages between the immersion stages, passes through a defined temperature program, in which the temperature does not exceed 70°C.

11. Process according to any one of the preceding claims, characterized in that the drying time of the individual drying stages between the immersion stages is about 1.5 minutes.

12. Process according to claim 1, characterized in that the immersion tool (14) together with the applied elastic material is, following the formation of the rolling ring (20), is subjected to a heat treatment according to a defined temperature progra, in which the maximum temperature does not exceed about 110°C.

13. Process according to claim 1, characterized in that the immersion tool (14) mounted suspended on a conveyor belt (3) is passed through the immersion baths (7, 9, 11) at a certain speed varying between 6 cm/s and 9 cm/s.

14. Process according to claim 1, characterized in that during the individual heat treatments, the immersion tool (14) is rotated around the longitudinal axis.

15. Process according to claim 14, characterized in that the rotation of the immersion tool (14) starts immediately after the complete removal from the immersion baths (7, 9, 11).

16. Process according to any one of the preceding claims, characterized in that the immersion tool (14) is passed through the heat treatment devices (8, 10, 12, 13, 13b) in the horizontal position.

17. Process according to one or several of the preceding claims, characterized in that the finished prophylactic is stripped from the immersion tool (14) by means of brushes rotating in opposite directions, with the help of at least one pressurized water jet (35).

18. Process according to claims 1 and 17, characterized in that the at least one water jet (35) assumes a certain angle relative to the longitudinal axis of the immersion tool (14) and acts on the front part of the immersion tool (14).

19. Device for producing a prophylactic, whereby the prophylactic consists of a shaped part made of thin-walled, elastic material, which is externally applied to the immersion tool (14) in immersion baths (7, 9, 11), and the immersion tool (14) consists of a cylindrical section (16) and a shaped section (17) and has in the shaped section (17) an annular groove (24) and a reservoir part (23, 31), and the immersion tool (14) is mounted on an endless conveyor belt (3),
characterized by
- three immersion tubs (7, 9, 11), in which a special mixture (latex) substantially consisting of natural rubber is present, whereby the first (7) of the three immersion tubs (7, 9, 11) is disposed substantially deeper relative to the endless conveyor belt (3) than the other two immersion tubs (9, 11);
- drying furnaces (8, 10, 13), for which provision is made in each case between the first and the second and the second and the third immersion tub, said furnaces being operated with a defined temperature profile and temperature program, in which the maximum temperature amounts to about 70°C; and
- that following the third immersion tub (11), provision is made first for a drying furnace (13), a brush device (13a) and another drying furnace (13b); and
- that it has a furnace (12) extending across the total length of the endless conveyor belt (3), said furnace being operated with a defined temperature profile and temperature program, whereby the maximum temperature does not exceed about 110°C;
- a rail (7a) extending along the immersion tub (7), said rail providing the longitudinal axis of the immersion tool (14) with a certain slanted position and putting the immersion tool (14) into rotation;
- a stripping device (6) having brushes rotating in opposite directions, and a pressurized water jet (35) acting on the front part of the immersion tool (14).

20. Device according to claim 19, characterized in that the immersion tool (14) is a long-stretched, preferably a glass cylinder closed at one end, said cylinder having on the surface within the closed end a certain contour with a deepening (ditch) (22).

21. Device according to claims 19 and 20, characterized in that the deepening (22) extends across about 30 mm, whereby the deepening (22) starts about 50 mm from the closed end (23) of the immersion tool (14).

22. Device according to claim 20, characterized in that the deepening (22) has, in the center, a straight part (21).

23. Device according to claims 19 and 20, characterized in that an annular groove (24) is worked into about the center of the straight part (21), with the opening angle of such groove varying between 60° and 120°.

24. Device according to any one of the preceding claims, characterized in that the annular groove (24) has no sharp points, corners and edges.

25. Device according to any one of the preceding claims, characterized in that the depth of the annular groove (24) varies between 0.3 and 1.5 mm.

26. Device according to any one of the preceding claims, characterized in that the speed of the conveyor belt during a production cycle is constant and amounts to about 8 cm/s.

27. Device according to claims 19 and 20, characterized in that the deepening (ditch)(22) extends over about 60 mm, whereby the deepening (22) starts about 40 mm from the closed end (23, 31) of the immersion tool (14).

28. Device according to claims 19, 20 and 25, characterized in that the deepening is composed of two approximately identical conical, truncated cone-like parts (28, 29), whereby the small cover surfaces of the truncated cones (28, 29) abut each other, forming in this way a sharp separation line (30).

29. Device according to claims 19, 20, 23 and 25, characterized in that the separation line (30) has an annular groove (24).

30. Device according to claim 28, characterized in that the jacket surfaces of the truncated cones (28, 29) are spherical.

31. Device according to claims 19 and 20, characterized in that the reservoir part (31) is formed on the top end of prophylactic in the shape of a club, whereby the narrower part of the reservoir (31) adjoins the glass piston.

32. Device according to any one of the preceding claims, characterized in that the diameter of the separation line (30) varies between 16 and 30 mm.

33. Device according to claim 31, characterized in that the diameter of the narrower lower part of the reservoir (31) varies between 4 and 10 mm.

34. Device according to claim 31, characterized in that the diameter of the wider top part of the reservoir (31) is 12 mm, and the length of the reservoir (31) is 16 mm.

35. Device according to any one of the preceding claims, characterized in that the colors of the elastic material to be applied to the immersion tool (14) during the immersion stage are different in the individual immersion tubs (7, 9, 11).

36. Device according to any one of the preceding claims, characterized in that the stripping device for stripping off the finished prophylactic has brushes (32) rotating in opposite directions.

37. Device according to claims 19 and 36, characterized in that the stripping device has at least one water jet nozzle, the pressurized water jet of which is acting on the immersion tool (14) in the front part of the latter.

## Revendications

1. Procédé de production de préservatifs, le préservatif se composant d'un préformé en matériau élastique à paroi mince qui, dans des bacs à immersion, est appliqué extérieurement sur un outil d'immersion, et le préformé composé d'une pièce (16) cylindrique et d'une pièce moulée (17) et qui présente un anneau à la partie supérieure moulée (17), l'outil d'immersion (14) étant plongé avec sa partie effilée à des profondeurs très différentes dans le matériau (latex) liquide à appliquer, sachant qu'après chaque opération de plongée, il y a un traitement thermique.

2. Procédé selon la revendication 1,
**caractérisé en ce que**
avant la première opération l'outil d'immersion (14) est nettoyé et débarrassé des résidus qui se trouvent sur l'outil d'immersion (14) et la surface est préparée de telle sorte que le matériau élastique déposé sur l'outil d'immersion (14) puisse être facilement enlevé.

3. Procédé selon la revendication 1,
**caractérisé en ce que**
lors de la première opération de plongée avec un outil préformé d'immersion (14), seulement la partie préformée (17) plonge dans le premier bac à immersion (7) de telle sorte qu'au moins l'évidement (22) plonge entièrement dans le premier bac à immersion (7).

4. Procédé selon les revendications 1 et 3,
**caractérisé en ce que**
lors de la première opération de plongée, l'outil d'immersion (14) est guidé à travers le bac à immersion (7) avec son axe longitudinal dans un certain angle par rapport à la surface du matériau élastique à appliquer, tournant autour de son propre axe longitudinal.

5. Procédé selon la revendication 1,
**caractérisé en ce que**
l'outil d'immersion (14) avec le matériau élastique appliqué est soumis à une première opération de séchage (8).

6. Procédé selon la revendication 1,
**caractérisé en ce que**
après la première opération de séchage, il y a une deuxième opération d'immersion lors de laquelle l'outil d'immersion (14) est plongé le plus loin possible dans une deuxième cuve à immersion (9).

7. Procédé selon la revendication 1,
**caractérisé en ce que**
après la deuxième opération d'immersion, une deuxième opération (10) de séchage est effectuée.

8. Procédé selon la revendication 1,
**caractérisé en ce que**
après la deuxième opération de séchage, une troisième opération de plongée dans une troisième cuve (11) à immersion est réalisée lors de laquelle l'outil d'immersion (14) n'est pas beaucoup moins introduit dans une troisième cuve à immersion (11) que lors de la deuxième opération de plongée.

9. Procédé selon la revendication 1,
**caractérisé en ce que**
après la troisième opération de plongée (13) un séchage est réalisé à la suite duquel un bourrelet (20) est constitué au moyen de brosses (13a) tournant en sens inverse et ensuite une opération de séchage (13b) a lieu.

10. Procédé selon l'une ou plusieurs des revendications précédentes,
**caractérisé en ce que**
l'outil d'immersion (14) pendant les différentes opérations de séchage entre les opérations de plongée parcourt un certain programme de température, la température ne dépassant pas 70°C.

11. Procédé selon l'une des revendications précédentes,
**caractérisé en ce que**
la durée de séchage des différentes opérations de séchage entre les opérations de plongée dure environ 1,5 minutes.

12. Procédé selon la revendication 1,
**caractérisé en ce que**
après la formation du bourrelet (20), l'outil d'immersion (14) avec le matériau élastique appliqué est soumis à un traitement thermique conformément à un certain programme de température dont la température maximum ne dépasse pas 110°C env.

13. Procédé selon la revendication 1,
**caractérisé en ce que**
l'outil d'immersion (14) fixé suspendu sur un convoyeur à bande (3) se déplace entre les bacs à immersion (7, 9, 11) à une certaine vitesse qui varie entre 6 cm/s et 9 cm/s.

14. Procédé selon la revendication 1,
**caractérisé en ce que**
l'outil d'immersion (14) tourne autour de son axe longitudinal pendant les différents traitements thermiques.

15. Procédé selon la revendication 14,
**caractérisé en ce que**
la rotation de l'outil d'immersion (14) commence directement après l'enlèvement complet des bacs à immersion (7, 9, 11).

16. Procédé selon l'une des revendications précédentes
**caractérisé en ce que**
l'outil d'immersion (14) passe par les dispositifs de traitements thermiques (8, 10, 12, 13, 13b) en position horizontale.

17. Procédé selon l'une ou plusieurs revendications précédentes
**caractérisé en ce que**
le préservatif fini est retiré de l'outil d'immersion (14) au moyen de brosses tournant en sens inverse avec l'aide d'au moins un jet d'eau (35) alimenté par pression.

18. Procédé selon les revendications 1 et 17,
**caractérisé en ce que**
au moins un jet d'eau (35) se trouve dans un certain angle par rapport à l'axe longitudinal de l'outil d'immersion (14) et agit dans sa partie avant sur l'outil d'immersion (14).

19. Dispositif de production de préservatifs, le préservatif se composant d'une pièce moulée en matériau mince qui est appliqué extérieurement sur un outil d'immersion (14) dans des bacs à immersion (7, 9, 11) et l'outil d'immersion (14) se composant d'une partie cylindrique (16) et d'une partie moulée (17) qui présente une rainure (24) et une partie réservoir (23, 31) dans la partie moulée (17), et l'outil d'immersion (14) étant fixé à un convoyeur à bande (3) sans fin,
**caractérisé en ce que**
- trois cuves à immersion (7, 9, 11) dans lesquelles se trouve un mélange spécial (Latex), se composant principalement de caoutchouc naturel, sachant que la première (7) des trois cuves à immersion (7, 9, 11) est beaucoup plus basse par rapport au convoyeur à bande (3) sans fin que les deux autres cuves à immersion (9, 11);
- entre la première et la deuxième cuve à immersion ainsi qu'entre la deuxième et la troisième, des fours de séchage (8, 10, 13) sont respectivement prévus, ayant un certain profil de température et un programme de température dont la température maximum est environ de 70°C; et
- derrière la troisième cuve à immersion (11) on a prévu d'abord un four de séchage (13), un dispositif de brosses (13a) et un autre four de séchage (13b); et
- qui présente un four (12) s'étendant sur toute la longueur du convoyeur (3) sans fin, possédant un certain profil de température et un programme de température, la température maximum ne dépassant pas 110°C environ;
- un rail (7a) guidé le long de la cuve à immersion (7), qui donne à l'axe longitudinal de l'outil d'immersion (14) une certaine position inclinée et qui met en rotation l'outil d'immersion (14);
- un dispositif de raclage (6), les brosses tournant en sens inverse et un jet d'eau (35) alimenté par pression qui agit sur la partie avant de l'outil d'immersion (14).

20. Dispositif selon la revendication 19,
**caractérisé en ce que**
l'outil d'immersion (14) est un cylindre, de préférence en verre, très allongé, fermé à une extrémité, qui présente un évidement (cavité) (22) à la surface dans la zone de l'extrémité fermée.

21. Dispositif selon les revendications 15 et 20,
**caractérisé en ce que**
l'évidement (22) s'étend sur env. 30 mm, l'évidement (22) commençant environ 50 mm après l'extrémité fermée (23) de l'outil d'immersion (14).

22. Dispositif selon la revendication 20
**caractérisé en ce que**
l'évidement (22) présente une partie droite (21) au milieu.

23. Dispositif selon les revendications 19 et 20,
**caractérisé en ce que**
à peu près au milieu de la partie droite (21) il y a une rainure (24) dont l'angle d'ouverture varie entre 60° et 120°.

24. Dispositif selon l'une des revendications précédentes
**caractérisé en ce que**
la rainure (24) ne présente pas d'extrémités, ni d'arêtes, ni d'angles tranchants.

25. Dispositif selon l'une des revendications précédentes,
**caractérisé en ce que**
la profondeur de la rainure (24) varie entre 0,3 et 1,5 mm.

26. Dispositif selon l'une des revendications précédentes,
**caractérisé en ce que**
la vitesse du convoyeur est constante pendant un cycle de production et est de 8 cm/s.

27. Dispositif selon les revendications 19 et 20,
**caractérisé en ce que**
l'évidement (cavité) (22) s'étend sur env. 60 mm, l'évidement commençant env. 40 mm après l'extrémité fermée (23, 31) de l'outil d'immersion (14).

28. Dispositif selon les revendications 19, 20 et 25,
**caractérisé en ce que**
l'évidement est constitué par deux pièces presque identiques coniques, tronconiques (28, 29), les petites surfaces de recouvrement des cônes tronqués (28, 29) se heurtant mutuellement, ce qui forme une ligne de séparation (30) bien nette.

29. Dispositif selon les revendications 19, 20, 23 et 25,
**caractérisé en ce que**
la ligne de séparation (30) présente une rainure (24).

30. Dispositif selon la revendication 29,
**caractérisé en ce que**
que les surfaces d'enveloppe des troncs de cône (28, 29) sont bombées.

31. Dispositif selon les revendications 19 et 20,
**caractérisé en ce que**
la partie réservoir (31) est de forme lobée à l'extrémité supérieure du préservatif, la partie plus étroite du réservoir (31) faisant suite au ballon.

32. Dispositif selon l'une des revendications précédentes,
**caractérisé en ce que**
le diamètre de la ligne de séparation (30) varie entre 16 et 30 mm.

33. Dispositif selon la revendication 31,
**caractérisé en ce que**
le diamètre de la partie inférieure étroite du réservoir (31) varie entre 4 et 10 mm.

34. Dispositif selon la revendication 31,
**caractérisé en ce que**
le diamètre de la partie supérieure plus large du réservoir (31) est de 12 mm et la longueur du réservoir (31) de 16 mm.

35. Dispositif selon l'une des revendications précédentes,
**caractérisé en ce que**
les coloris du matériau élastique à appliquer sur l'outil d'immersion (14) pendant l'opération de plongée sont différents dans chacune des cuves à immersion (7, 9, 11).

36. Dispositif selon l'une des revendications précédentes,
**caractérisé en ce que**
le dispositif de raclage pour retirer le préservatif fini présente des brosses (32) tournant en sens inverse.

37. Dispositif selon les revendications 19 et 36,
**caractérisé en ce que**
le dispositif de raclage présente au moins une buse de jet d'eau, le jet d'eau alimenté par pression dans la partie avant de l'outil d'immersion (14) agissant sur l'outil d'immersion.
